(19) Europäisches Patentamt
European Patent Office
Office européen des brevets

(11) Publication number: **0 075 365**
**A2**

(12) **EUROPEAN PATENT APPLICATION**

(21) Application number: **82201141.7**

(22) Date of filing: **15.09.82**

(51) Int. Cl.³: **C 07 D 295/14**, C 07 C 121/45, C 07 C 101/34, C 07 F 15/00

(30) Priority: **23.09.81 IT 2408781**

(43) Date of publication of application: **30.03.83**
**Bulletin 83/13**

(84) Designated Contracting States: **BE DE FR GB NL SE**

(71) Applicant: **ANIC S.p.A., Via Ruggero Settimo, 55, I-90139 Palermo (IT)**

(72) Inventor: **Chiusoli, Gian Paolo, Via Solferino 18 bis, I-43100 Parma (IT)**
Inventor: **Costa, Mirco, Piazza A. Fontanesi 11, I-42100 Reggio Emilia (IT)**
Inventor: **Pallini, Luciano, Via V.Bottego 3, I-43045 - Fornovo Taro(Parma) (IT)**
Inventor: **Terenghi, Giuliana, Via C. Collodi 18 I-42024 Castelnovo di Sotto, Reggio Emilia (IT)**

(74) Representative: **Roggero, Sergio et al, Ing. Barzanò & Zanardo Milano S.p.A. Via Borgonuovo 10, I-20121 Milano (IT)**

(54) **Process for preparing tertiary amines with a tridecatrienyl chain, and the compounds obtained.**

(57) Compounds are described of formula $R^IR^{II}NCH_2CH=CHCH_2CR^{III}R^{IV}CH_2CR=CHCH_2CH_2CH_2CR=CH_2$ in which R is hydrogen or an alkyl radical containing up to 5 carbon atoms, $R^I$ and $R^{II}$ are alkyl radicals containing up to 12 carbon atoms, possibly linked together to form a cycle either containing or not containing heteroatoms, and $R^{III}$ and $R^{IV}$ can be $-COOR^V$, $COR^V$ or CN where $R^V$ is an alkyl radical containing up to 5 carbon atoms.

The preparation is carried out by reacting a conjugate diene of formula $CH_2=C(R)CH=CH_2$ with amines of formula $R^IR^{II}NH$ and vinylcyclopropanes of formula

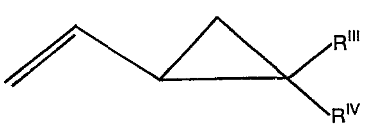

in the presence of particular palladium complexes of formula

$[Pd(PR_3^{VI})_nL_m]$

in which n can assume any value between 1 and 4, m can be 0 or vary up to 3, and n+m is 3 or 4, $PR_3^{VI}$ are trialkyl or triaryl phosphines where $R^{VI}$ is a hydrocarbon radical containing up to 13 carbon atoms, and L is an olefine binder containing up to 20 carbon atoms.

EP 0 075 365 A2

"PROCESS FOR PREPARING TERTIARY AMINES WITH A TRIDECATRIENYL CHAIN, AND THE COMPOUNDS OBTAINED"

This invention relates to compounds of new conception, namely tertiary amines with a tridecatrienyl chain, of formula

$$R^{I}R^{II}NCH_2CH{=}CHCH_2CR^{III}R^{IV}CH_2CR{=}CHCH_2CH_2CH_2CR{=}CH_2$$

in which R is hydrogen or an alkyl radical containing up to 5 carbon atoms, $R^{I}$ and $R^{II}$ are alkyl radicals containing up to 12 carbon atoms, possibly linked together to form a cycle which either contains or does not contain heteroatoms, and $R^{III}$ and $R^{IV}$ can be $-COOR^{V}$, $COR^{V}$ or CN where $R^{V}$ is an alkyl radical containing up to 5 carbon atoms.

The said compounds, which are useful as intermediates in organic synthesis or as additives in polymer formulation, are prepared by a process which constitutes a further subject matter of the present invention, and in which a conjugate diene of formula

$$CH_2{=}C(R)CH{=}CH_2$$

is reacted with amines of formula

$$R^{I}R^{II}NH$$

and vinylcyclopropanes of formula

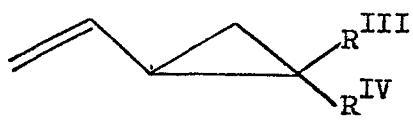

the reaction being carried out in the presence of phosphine complexes of transition metals, particularly of palladium complexes of the following formula

$$[Pd(PR_3^{VI})_nL_m]$$

in which n can assume any value between 1 and 4, m can be 0

or vary up to 3, and n+m is 3 or 4, $PR_3^{VI}$ are trialkyl or triaryl phosphines where $R^{VI}$ is a radical containing up to 13 carbon atoms, and L is an olefin binder containing up to 20 carbon atoms, for example dibenzalacetone or cyclooctadiene.

The reaction is carried out at a temperature of between 0 and 100°C in an inert gas atmosphere in the presence of a solvent, generally a nitrile compound or an alcohol, having a boiling point less than 120°C.

It is surprising that although the reactants are introduced together, the four molecules become arranged in a precise order, and the amine only partly gives the product resulting from attachment to the diene without reacting with the vinylcyclopropane.

To prevent this secondary reaction, it is in any case sufficient to add the diene later, after the amine and vinylcyclopropane (in the ratio of 1:1) have reacted, and without treating the reaction mixture in any way.

The reaction is carried out by simply bringing the reactants and catalyst into contact under nitrogen. The separation is carried out by conventional methods (separation by high vacuum distillation, gas chromatography, liquid chromatography or column chromatography).

The starting substances are prepared simply from dichloro or dibromo butene and $CH_2R^{III}R^{IV}$ compounds.

The reactants are used either in stoichiometric ratios or in excess (1-3 moles of amine per mole of vinylcyclopropane,

2-10 moles of butadiene per mole of vinylcyclopropane) according to the particular conditions used in relation to the reacted substrates.

The number of moles of reactants which can be made to react per mole of complex can reach high values, for example by increasing the reaction time.

The aforesaid operating methods will be apparent from the following non-limiting examples of the invention.

EXAMPLE 1

0.022 g (0.019 mM) of Pd $(PPh_3)_4$ are fed into a 25 ml flask under a nitrogen atmosphere. 0.4 g (1.9 mM) of 1,1-dicarbethoxy-2-vinylcyclopropane in 3 ml of acetonitrile are then added, followed by 0.5 ml (5.7 mM) of morpholine and, on cooling with an ice and salt bath, 1.7 ml (19.5 mM) of 1,3-butadiene. The reaction mixture is then heated by an oil bath to 70°C for 40 hours. It is allowed to cool, and the unreacted butadiene, the solvent and the lighter products deriving from the attachment of the morpholine to two molecules of butadiene are removed under vacuum. On distilling with a bulb furnace and purifying the obtained residue by column chromatography, 0.61 g of product are obtained consisting mainly of

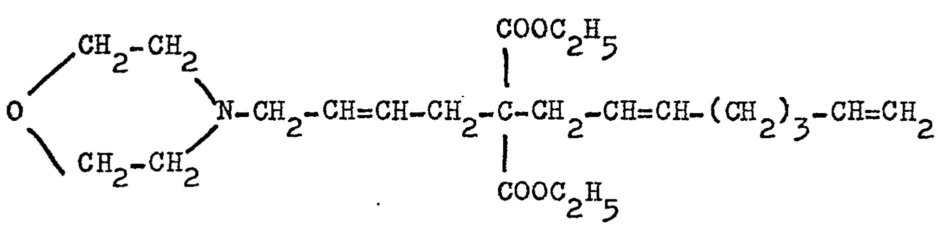

corresponding to a yield of 79% with respect to the fed

vinylcyclopropane, and a catalytic effectiveness of 79 moles of product per mole of palladium complex.

Analogous results are obtained after some days at ambient temperature, or by carrying out the reaction in stages, and in particular by reacting 1,1-dicarbethoxy-2-vinylcyclopropane with morpholine in the presence of Pd $(PPh_3)_4$, which after 24 hours at ambient temperature gives the corresponding N-(5,5-dicarbethoxy-2-pentenyl)-morpholine, to which 1,3-butadiene is then added.

EXAMPLE 2

Under the same conditions as example 1, 0.418 g of 1,1-dicarbomethoxy-2-vinylcyclopropane (2.27 mM), 0.6 ml of morpholine (6.9 mM), and 2 ml of 1,3-butadiene (23 mM) in 3.5 ml of acetonitrile are reacted in the presence of 0.026 g of Pd $(PPh_3)_4$ (0.0225 mM). On proceeding as in example 1, after 20 hours at 70°C, 0.645 g of a product are obtained which consists mainly of:

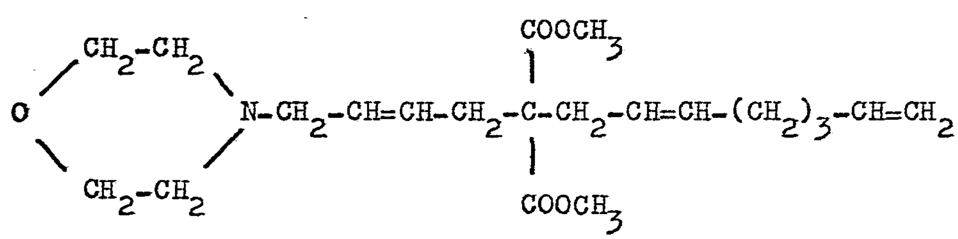

corresponding to a yield of 75% with respect to the fed vinylcyclopropane, and a catalytic effectiveness of 75 moles of product per mole of palladium complex.

EXAMPLE 3

0.024 g of Pd $(PPh_3)_4$ (0.0208 mM) are fed into a 25 ml flask under a nitrogen atmosphere. 0.350 g of 1-cyano-1-carbethoxy-2-vinylcyclopropane (2.1 mM) and 0.66ml of diethylamine (6.4 mM)

in 5 ml of acetonitrile are then added. 2 ml of 1,3-butadiene (23 mM) are added after 20 hours at ambient temperature. After 24 hours, the unreacted butadiene is removed, and the solvent and lighter products (deriving from the attachment of the diethylamine to two molecules of butadiene) are distilled off, to give a residue which after column chromatography purification gives 0.67 g of a product consisting mainly of:

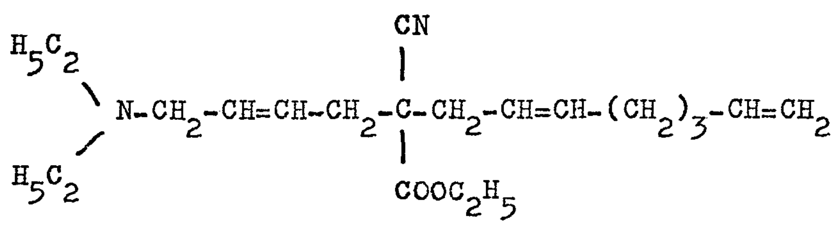

corresponding to a yield of 91% with respect to the fed vinylcyclopropane, and a catalytic effectiveness of 91 moles of product per mole of palladium complex.

Analogous results are obtained after a week at ambient temperature by carrying out the reaction in a single stage.

The reaction was also carried out using 5 ml of ethyl alcohol as solvent, employing the same procedure as indicated in this example.

0.68 g of the initially obtained tertiary amine were obtained.

EXAMPLE 4

0.031 g of $Pd(PPh_3)_4$ (0.0269 mM), 0.506 g of 1-acetyl-1-carbethoxy-2-vinylcyclopropane (2.8 mM), 0.83 ml of diethylamine (8.0 mM) in 6 ml of acetonitrile and 2 ml of 1,3-butadiene (23 mM) are reacted under the same conditions as example 3. On operating as in example 3, 0.94 g of a product are obtained consisting mainly of:

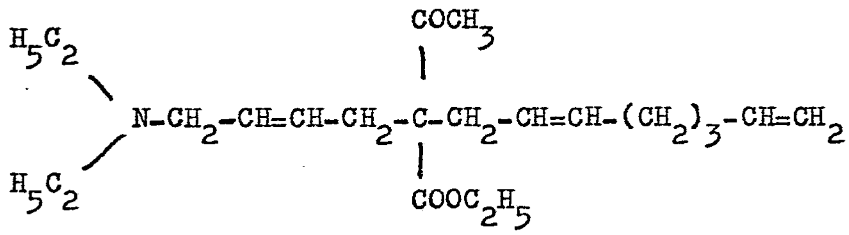

corresponding to a yield of 93% with respect to the fed vinylcyclopropane, and a catalytic effectiveness of 93 moles of product per mole of palladium complex.

EXAMPLE 5

0.12 g of $Pd(PPh_3)_4$ (0.104 mM), 1.9 g of 1,1-dicarbomethoxy-2-vinylcyclopropane (10.3 mM), 1.8 ml of morpholine (20.6 mM), 5 ml of isoprene (50 mM) in 15 ml of acetonitrile are reacted in a 50 ml flask under a nitrogen atmosphere for four days at 70°C.

After separating the palladium by reduction with CO, the lighter products (products resulting from the attachment of the amine to the isoprene dimers and the attachment of the amine to the vinylcyclopropane) are removed by distillation under vacuum, to give a residue of 2.32 g consisting mainly of:

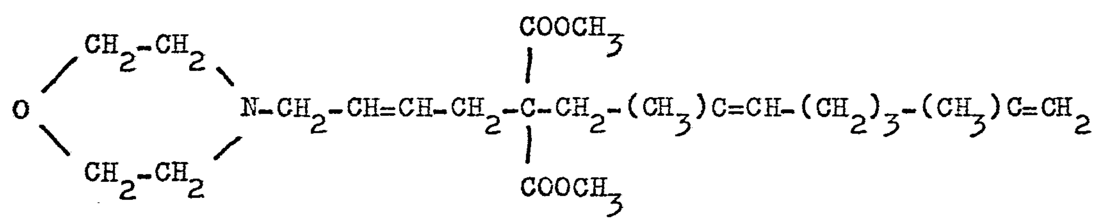

The quantity of product obtained corresponds to a yield of 55% with respect to the fed vinylcyclopropane, and to a catalytic effectiveness of 55 moles of product per mole of palladium complex.

Analogous results are obtained by using diethylamine instead of morpholine, and 1,1-dicarbethoxy-2-vinylcyclopropane

instead of 1,1-dicarbomethoxy-2-vinylcyclopropane.

EXAMPLE 6

By reacting $Pd(PPh_3)_4$ with 1-cyano-1-carbethoxy-2-vinylcyclopropane and diethylamine in acetonitrile in a nitrogen atmosphere for 24 hours at ambient temperature and then heating to 80°C for a further 24 hours after adding isoprene, the following compound is obtained as the main product of the successive attachment of the amine to the vinylcyclopropane and to the isoprene:

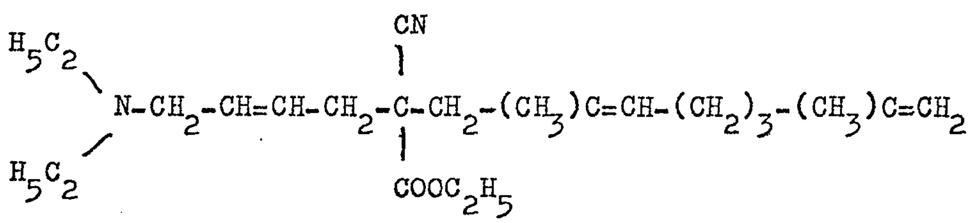

CLAIMS:

1. Tertiary amines with a tridecatrienyl chain of formula

$$R^IR^{II}NCH_2CH=CHCH_2CR^{III}R^{IV}CH_2CR=CHCH_2CH_2CH_2CR=CH_2$$

in which R is hydrogen or an alkyl radical containing up to 5 carbon atoms, $R^I$ and $R^{II}$ are alkyl radicals containing up to 12 carbon atoms, possibly linked together to form a cycle either containing or not containing heteroatoms, and $R^{III}$ and $R^{IV}$ can be $-COOR^V$, $COR^V$ or CN where $R^V$ is an alkyl radical containing up to 5 carbon atoms.

2. A process for preparing compounds as claimed in claim 1, comprising reacting a conjugate diene of formula $CH_2=C(R)CH=CH_2$ with amines of formula

$$R^IR^{II}NH$$

and vinylcyclopropanes of formula

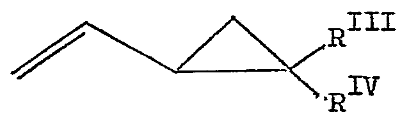

in the presence of phosphine complexes of transition metals.

3. A process as claimed in the preceding claim, characterised in that the reaction is carried out preferably in the presence of a palladium complex of formula

$$[Pd(PR_3{}^{VI})_nL_m]$$

in which n can assume any value between 1 and 4, m can be 0 or vary up to 3, and n+m is 3 or 4, $PR_3{}^{VI}$ are trialkyl or triaryl phosphines where $R^{VI}$ is a hydrocarbon radical containing up to 13 carbon atoms, and L represents an olefin binder containing up to 20 carbon atoms.

4. A process as claimed in claim 2 or 3, characterised in that the reaction is carried out at a temperature of between 0 and 100°C.

5. A process as claimed in claim 2 or 3, characterised in that the reaction is carried out in a solvent chosen from nitrile compounds or alcohols.

6. A process as claimed in claim 2 or 3, characterised in that the reaction is carried out under an inert gas atmosphere.